(19) Europäisches Patentamt
European Patent Office
Office européen des brevets

(11) **EP 4 169 446 A1**

(12) **EUROPEAN PATENT APPLICATION**

(43) Date of publication:
**26.04.2023 Bulletin 2023/17**

(21) Application number: **22202345.9**

(22) Date of filing: **19.10.2022**

(51) International Patent Classification (IPC):
**A61B 5/287** (2021.01)    **A61B 5/318** (2021.01)
**A61B 5/349** (2021.01)    **A61B 5/361** (2021.01)
**A61B 5/363** (2021.01)    **A61B 5/00** (2006.01)

(52) Cooperative Patent Classification (CPC):
**A61B 5/318; A61B 5/287; A61B 5/349;**
**A61B 5/361; A61B 5/363; A61B 5/7264**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.10.2021 US 202117506120**

(71) Applicant: **Biosense Webster (Israel) Ltd.
Yokneam 2066717 (IL)**

(72) Inventors:
- **COHEN, Benjamin**
  **2066717 Yokneam (IL)**
- **KATZ, Natan Sharon**
  **2066717 Yokneam (IL)**
- **TURGEMAN, Aharon**
  **2066717 Yokneam (IL)**
- **DVORKIN, Vladimir**
  **2066717 Yokneam (IL)**

(74) Representative: **Carpmaels & Ransford LLP
One Southampton Row
London WC1B 5HA (GB)**

(54) **CLUSTERING OF ELECTROPHYSIOLOGICAL (EP) SIGNALS USING SIMILARITIES AMONG ARRHYTHMOGENIC ACTIVATIONS**

(57) A method includes defining multiple different types of arrhythmias. Similarity measures are defined for the types of arrhythmias. A set of EP signals is received, the set acquired in a heart of a patient. Using the similarity measures, the set of EP signals is partitioned into at least two clusters, each cluster containing EP signals complying with a respective similarity measure for a respective type of arrhythmia. The clusters are indicated to a user.

EP 4 169 446 A1

**Description**

**FIELD OF THE INVENTION**

The present invention relates generally to electrophysiological (EP) signal analysis, and particularly to the analysis of cardiac arrhythmias.

**BACKGROUND OF THE INVENTION**

Clustering refers to the task of identifying groups or clusters in a data set. In density-based clustering, a cluster is a set of data objects spread in the data space over a contiguous region of a high density of objects. Density-based clusters are separated from each other by contiguous regions of a low density of objects. Data objects located in low-density regions are typically considered noise or outliers.

Methods to characterize the propagation of activation signals in cardiac tissue were previously proposed in the patent literature. For example, U.S. Patent No. 9,737,227 describes a system and method for mapping a cardiac anatomical structure that includes sensing activation signals of physiological activity with a plurality of mapping electrodes disposed in or near the anatomical structure. Patterns among the sensed activation signals are identified based on a similarity measure generated between each unique pair of identified patterns which are classified into groups based on a correlation between the corresponding pairs of similarity measures. A characteristic representation is determined for each group of similarity measures and displayed as a summary plot of the characteristic representations.

As another example, U.S. Patent Application Publication No. 2010/0280400 describes a cardiac rhythm management system that can be used to detect episode beats associated with cardiac events in a subject's body. These events may be monitored and depolarization morphology information can be derived for candidate arrhythmic beats in an arrhythmia episode. An arrhythmic beat morphology template may be formed from selecting at least one of the candidate arrhythmic beats based upon user's labeling according to specific morphologies of one or more candidate episodes. Methods of use are also presented.

U.S. Patent Application Publication No. 2004/0176697 describes an analysis of electrocardiograms (ECGs) during atrial fibrillation. In particular, the invention relates to the use of such methods for the creation and validation of cardiac models and use in the refined diagnosis of heart disease. Classification of the atrial fibrillation of a given patient or group of patients is performed using an auto regressive (AR) model with coefficients subjected to a mathematical cluster analysis, including hierarchical methods (e.g., single linkage, average linkage (weighted and unweighted), centroid, median and complete linkage) and non-hierarchical methods ,e.g., the k-means clustering algorithm, adaptive k-means, k-medoids, and fuzzy clustering.

**SUMMARY OF THE INVENTION**

An embodiment of the present invention that is described hereinafter provides a method for clustering of electrophysiological signals using similarities among arrhythmogenic activations, including defining multiple different types of arrhythmias. Similarity measures are defined for the types of arrhythmias. A set of EP signals is received, the set acquired in a heart of a patient. Using the similarity measures, the set of EP signals is partitioned into at least two clusters, each cluster containing EP signals complying with a respective similarity measure for a respective type of arrhythmia. The clusters are indicated to a user.

In some embodiments, indicating the clusters includes presenting a representative EP signal of each cluster.

In some embodiments, partitioning the set of EP signals includes assigning the EP signals to the clusters based on a number, N, of activations in each EP signal over a given window of interest.

In an embodiment, partitioning the set of EP signals includes assigning the EP signals to the clusters based on a number of cross iso-peaks within an activation in each EP signal.

In another embodiment, partitioning the set of EP signals includes assigning the EP signals to the clusters based on a width of an activation in each EP signal.

In some embodiments, partitioning the set of EP signals includes assigning the EP signals to the clusters based on a peak-to-peak bi-polar voltage of an activation in each EP signal.

In some embodiments, partitioning the set of EP signals includes assigning the EP signals to the clusters based on a sharpest slope of an activation in each EP signal.

In an embodiment, the similarity measure is one of a linear sum function and a quadratic sum function.

In some embodiments, the EP signals are electrograms.

There is additionally provided, in accordance with another embodiment of the present invention, a system for clustering of electrophysiological signals using similarities among arrhythmogenic activations, including a memory and a processor. The memory is configured to store (i) definitions multiple different types of arrhythmias, and (ii) definitions of similarity measures for the types of arrhythmias. The processor is configured to (a) receive a set of EP signals acquired in a heart of a patient, (b) using the similarity measures, partition the set of EP signals into at least two clusters, each cluster containing EP signals complying with a respective similarity measure for a respective type of arrhythmia, and (c) indicate the clusters to a user.

## BRIEF DESCRIPTION OF THE DRAWINGS

The present invention will be more fully understood from the following detailed description of the embodiments thereof, taken together with the drawings in which:

Fig. 1 is a schematic, pictorial illustration of a system for electrophysiological (EP) mapping and ablation, in accordance with an exemplary embodiment of the present invention;
Fig. 2 is a set of schematic graphs of EP signals, each graph representing a different type of cardiac EP signal, in accordance with an exemplary embodiment of the present invention; and
Fig. 3 is a flow chart that schematically illustrates a method of clustering EP signals using similarities among arrhythmogenic EP signals, in accordance with an exemplary embodiment of the present invention.

## DETAILED DESCRIPTION OF EMBODIMENTS

OVERVIEW

The electrical activity of tissue of an organ of a patient, such as a cardiac chamber, can be mapped ,e.g., electroanatomically and/or electrophysiologically, using a mapping catheter having suitable electrodes fitted at its distal end. An EP mapping system may acquire and analyze the signals output by the catheter.

In practice, during a cardiac arrhythmia analysis procedure, a very large number, for example, up to few tens of thousands or even more EP signals (e.g., bi-polar electrograms, may be acquired. Within these numerous EP signals, a physician may be interested in looking only at a small subset of the EP signals, for example electrograms showing some special fractionation, or electrograms characterized by a particular cycle length or local activation time (LAT). However, because of the large number of acquired EP signals, it is both difficult and time consuming to locate members of a subset among the entire set of acquired EP signals.

Embodiments of the present invention that are described hereinafter provide algorithms that automatically divide a set of EP signals into clusters (EP signals may be electrograms (EGM) or electrocardiograms (ECGs), for example). In an exemplary embodiment, a set of electrograms is acquired from a patient with arrhythmia. The electrograms are then analyzed to find parameters such as activation times, activation duration, number of peaks in an electrogram, slopes of the peaks, and the like. An algorithm uses these parameters to associate each electrogram with one of multiple types of predefined EP activations, such as normal conduction, atrial fibrillations, flutter, tachycardia, or other type of arrhythmia.

For each type of EP signal, a "similarity measure" is predefined, being the distance relating EP signals (e.g., electrograms) within the defined type of EP signal (e.g., type of arrhythmia). This mathematical similarity measure, for example one defined by a similarity metric as described below, delineates the bounds of the parameters defining "neighboring" signals. As an example, electrograms for a particular type of arrhythmia may be defined as those having two activations, each peak having a maximum voltage within some specified range, e.g., between 2 mV and 5 mV. An electrogram is deemed in the neighborhood of a given electrogram of this type if its two activations are within a given similarity difference (e.g., 10%) of those of the given electrogram.

In some embodiments, a processor runs a clustering algorithm that uses the predefined types and their respective distances to decide if a particular EP signal is clustered within one of the defined types. Typical clustering algorithms that may be used include the DBSCAN and the OPTICS algorithms, which were previously described in the literature. The two algorithms are described, for example, in a review paper entitled "Density-based clustering," by Kriegel et al., WIREs Data Mining and Knowledge Discovery, Volume 1, Issue 3, May/June 2011, pages 231-240 of, which is incorporated herein by reference.

In some embodiments, the processor clusters the EP signals automatically without user intervention. Subdividing the EP signals into clustered groups (i.e., types) of signals, for example ten groups, reduces the amount of time a physician may need to examine the EP signals. Thus, the physician may only need to inspect ten cluster-type representative signals, rather than many or all of the acquired signals.

SYSTEM DESCRIPTION

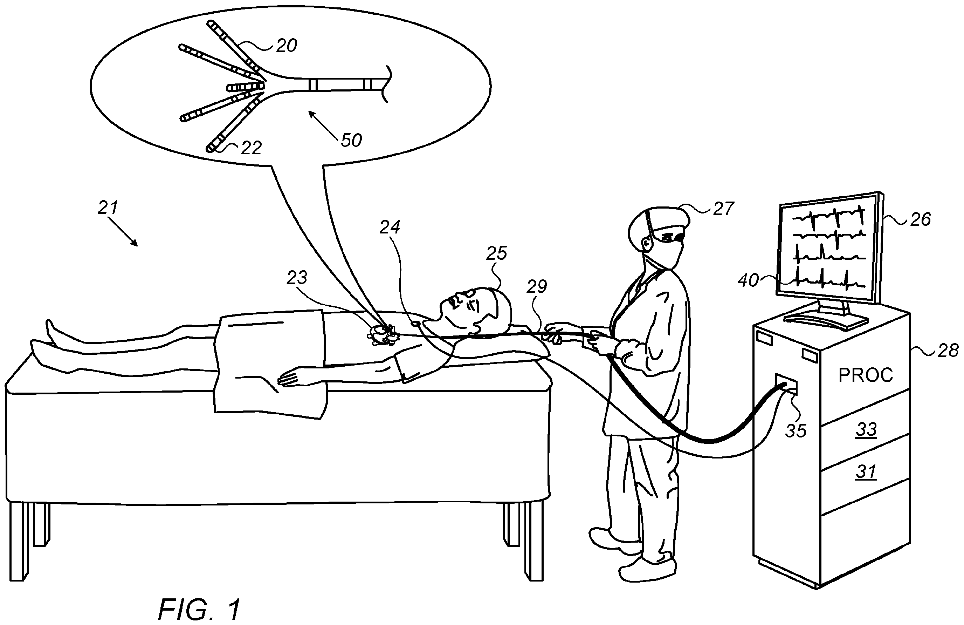

Fig. 1 is a schematic, pictorial illustration of a system 21 for electrophysiological (EP) mapping and ablation, in accordance with an embodiment of the present invention. Fig. 1 depicts a physician 27 using an EP mapping catheter 29 to perform an EP mapping of a heart 23 of a patient 25. Catheter 29 comprises, at its distal end, a multi-channel electrode array 50 comprising one or more arms 20, each of which is coupled to mapping-electrodes 22. During the mapping procedure, electrodes 22 acquire and/or inject signals from and/or to the tissue of heart 23. In particular, electrodes 22 acquire intracardiac EP signals, such as unipolar and/or bipolar electrograms.

Catheter 29 may be further used to perform an ablation, such as a radiofrequency (RF) or irreversible electroporation (IRE).

The respective locations of mapping-electrodes 22 inside heart 23 (i.e., where the electrograms are measured) are also tracked, such that a processor 28 may link each acquired electrogram with the location at which the signal was acquired. System 21 externally senses electrical position signals using a plurality of external electrodes 24 coupled to the body of patient 25; for example, three external electrodes 24 may be coupled to the patient's chest, and another three external electrodes may be coupled to the patient's back. For ease of illustration, only one external electrode is shown in Fig. 1.

An example of a system capable of using the sensed electrical position signals to track the locations of mapping-electrodes 22 inside heart 23 of the patient is the CARTO®3 system (produced by Biosense Webster, Irvine, California). The CARTO®3 system uses a tracking method known as Advanced Current Location (ACL), which is described in detail in U.S. Patent No. 8,456,182 whose disclosure is incorporated herein by reference.

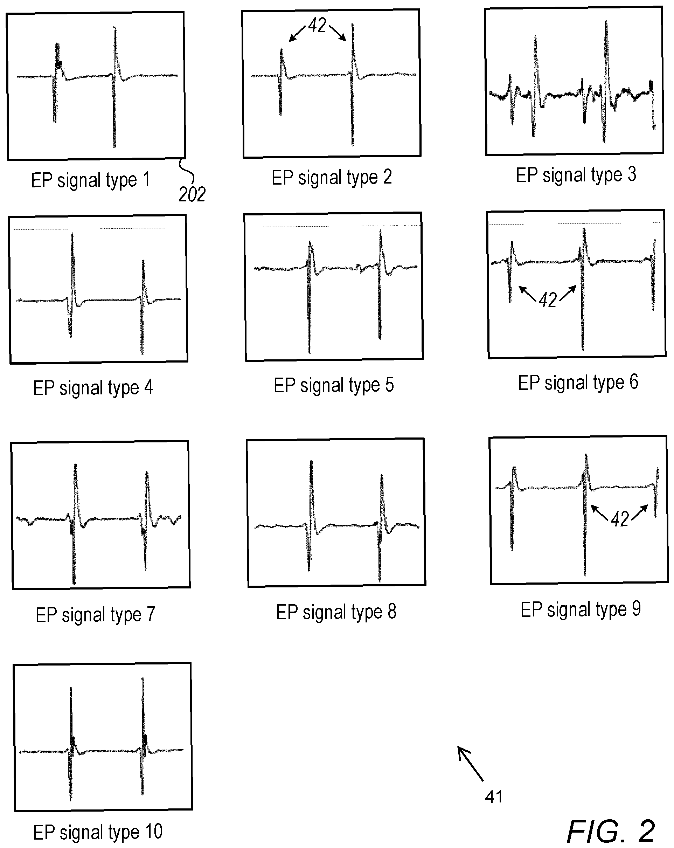

A data acquisition module 33 receives the multiple electrograms conveyed to an electrical interface 35 over a wire link that runs in catheter 29. Processor 28 of system 21 receives these cardiac signals via the electrical interface 35, and uses the disclosed clustering algorithm based on the aforementioned similarity function to cluster these signals according to different predefined arrhythmia types, e.g., atrial fibrillations, tachycardias, flutter and more, which may amount, as in the example shown in Fig. 2, to about ten EP signal types (e.g., types of electrograms 40 shown on a display 26 of system 21). In this way physician 27 may need to inspect only a small number (e.g., ten) of the EP signals (e.g., signals 40), rather than many more of the signals acquired, in order to clinically assess the mapping results. Based on the information, physician 27 may, for example, decide to perform an ablation.

The definitions of the various types of arrhythmia, and the corresponding definitions of the similarity measures, are typically stored in a memory 31 for use by processor 28. Processor 28 also stores the clustered electrograms in memory 31 for further analysis, such as for constructing an EP map.

The exemplary embodiment shown in Fig. 1 is chosen purely for the sake of conceptual clarity. Other types of multi-electrode sensing geometries, such as of the Lasso® catheter (produced by Biosense Webster) or a basket catheter may also be employed. Additionally, contact sensors may be fitted at the distal end of electro-anatomical catheter 29 and transmit data indicative of the physical quality of electrode contact with tissue. In an exemplary embodiment, measurements of some electrodes 22 may be discarded because their physical contact quality is poor, and the measurements of other electrodes may be regarded as valid because their contact quality is high.

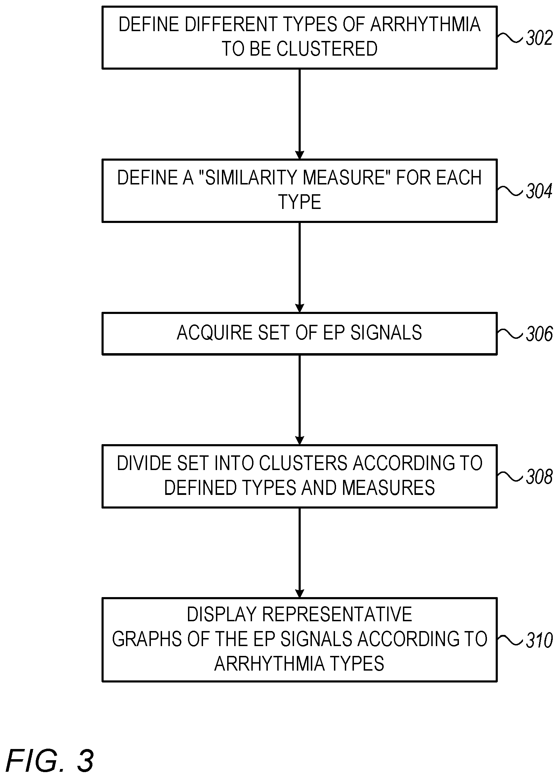

Processor 28 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 28 runs a dedicated algorithm that enables processor 28 to perform the steps described in Fig. 3.

CLUSTERING OF EP SIGNALS USING SIMILARITIES AMONG ARRHYTHMOGENIC ACTIVATIONS

As noted above, a clustering algorithm that is provided analyzes acquired EP signals to find parameters of the EP signals such as activation times, activation duration, number of peaks in an EP signal, and slopes of the peaks. The processor utilizes an algorithm that uses these parameters in a similarity measure to associate each EP signal, if found aberrative, with different predefined arrhythmia types.

In an exemplary embodiment, by-type clustering parameters for atrial flutter arrhythmia (for the similarity function) are:

Number of activations within the window of interest (WOI): N
Number of cross iso-peaks within an activation: P
Width of an activation: W
Peak to peak bipolar voltage of an activation: V
Sharpest slope of an activation: S

Between a pair of EP signals, a normalized value of the similarity function within the range of [0,1] is correspondingly calculated using the differences: dP, dW, dV, dS.

Assuming a stage clustering model, a first stage is characterized solely by a number N of activations within the WOI, and produce four clusters (flat: 0 activations; single potential, double potential, other: more than two activations).

The second stage of the model is calculated based on a similarity function *F(EP_signal_1, EP_signal_2)* on the rest of the parameters:

$$F(EP\_sig\_1, EP\_sig\_2)=0.5 \cdot dP+0.25 \cdot dS+0.125 \cdot dV+0.125 \cdot dW$$

The above linear similarity function is brought by way of example. Other functions, or norms, for example, quadratic ones in dP, dW, dV, and dS, may be used.

Fig. 2 is a set 41 of schematic graphs of EP signals, each graph representing a different cardiac activation type, in accordance with an exemplary embodiment of the present invention. Fig. 2 shows ten such schematic graphs representative of respective predefined clusters of cardiac EP signal types, such as types of arrhythmogenic activations. The EP signals shown are bipolar electrograms.

In set 41, different graphs may have a different number of activations 42, N, within a common WOI 202. For example, EP signals types 2 and 4 have N=2 activations 42 (e.g., peaks, complexes), whereas EP signals types 6 and 9 have N=3 peaks. In that regard, EP signal type 3 is unique by having N>4 peaks.

As another example, EP signals type 7 shows a large width of an activation, W, whereas EP signals types 4 and 10 show a narrower W.

A physician may need to inspect only set 41 of the ten-cluster representative EP signals types 1 to 10 in order to assess the cardiac activity and to determine if and which of the types has clinical significance. For example, most types may represent normal cardiac activities with only a small number of types representing suspicious electrical activity, abnormal substrate, possible ablation target, or other morphology of interest to a physician.

The graphs of Fig. 2 are schematic and brought purely by way of example to demonstrate the technique. In particular, set 41 of the shown graphs is not intended to represent a particular use case (e.g., to represent a diagnostic value case). Actual EP signals of actual arrhythmias may vary from the ones shown here.

Fig. 3 is a flow chart that schematically illustrates a method of clustering EP signals using similarities among arrhythmogenic EP signals, in accordance with an exemplary embodiment of the present invention. The algorithm, according to the presented exemplary embodiment, carries out a process that begins when a user, such as physician 27, defines, (e.g., selects using processor 28 with installed algorithm) a group of different arrhythmia types, at an arrhythmia type defining step 302.

Next, the algorithm selects a similarity measure defined for each arrhythmia type, with physician 27 optionally adjusting parameters of the selected one or more similarity measures, at similarity measures defining step 304.

At an EP data receiving step 306, processor 28 receives a set of thousands of EP signals acquired by a multi-electrode catheter (e.g., catheter 29) that mapped (or is mapping in real-time) a cardiac chamber.

Using the one or more similarity measures, at a clustering step 308, processor 28 partitions the set of EP signals into at least two clusters, each cluster characterized by its EP signals complying with the respective similarity measure for the type of arrhythmia.

Finally, processor 28 presents on display 26 representative graphs, such as set 41, of the different clusters, at EP signals presentation step 310. Typically, the processor selects a median signal from each cluster of signals as a representative signal for the cluster. Alternatively, however, any other suitable selection can be used.

Although the embodiments described herein mainly address cardiac applications, the methods and systems described herein can also be used in other applications, such as in mapping of electrical activity in the brain.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1. A method for clustering of electrophysiological signals using similarities among arrhythmogenic activations, the method comprising:

   defining multiple different types of arrhythmias;

defining similarity measures for the types of arrhythmias;
receiving a set of EP signals acquired in a heart of a patient;
using the similarity measures, partitioning the set of EP signals into at least two clusters, each cluster containing EP signals complying with a respective similarity measure for a respective type of arrhythmia; and
indicating the clusters to a user.

2. The method according to claim 1, wherein indicating the clusters comprises presenting a representative EP signal of each cluster.

3. The method according to claim 1, wherein partitioning the set of EP signals comprises assigning the EP signals to the clusters based on a number, N, of activations in each EP signal over a given window of interest.

4. The method according to claim 1, wherein partitioning the set of EP signals comprises assigning the EP signals to the clusters based on a number of cross iso-peaks within an activation in each EP signal.

5. The method according to claim 1, wherein partitioning the set of EP signals comprises assigning the EP signals to the clusters based on a width of an activation in each EP signal.

6. The method according to claim 1, wherein partitioning the set of EP signals comprises assigning the EP signals to the clusters based on a peak-to-peak bi-polar voltage of an activation in each EP signal.

7. The method according to claim 1, wherein partitioning the set of EP signals comprises assigning the EP signals to the clusters based on a sharpest slope of an activation in each EP signal.

8. A system for clustering of electrophysiological signals using similarities among arrhythmogenic activations, the system comprising:

  a memory configured to store:

    definitions multiple different types of arrhythmias; and
    definitions of similarity measures for the types of arrhythmias; and

  a processor, which is configured to:

    receive a set of EP signals acquired in a heart of a patient;
    using the similarity measures, partition the set of EP signals into at least two clusters, each cluster containing EP signals complying with a respective similarity measure for a respective type of arrhythmia; and
    indicate the clusters to a user.

9. The system according to claim 8, wherein the processor is configured to indicate the clusters by presenting a representative EP signal of each cluster.

10. The system according to claim 8, wherein the processor is configured to partition the set of EP signals by assigning the EP signals to the clusters based on a number, N, of activations in each EP signal over a given window of interest.

11. The system according to claim 8, wherein the processor is configured to partition the set of EP signals by assigning the EP signals to the clusters based on a number of cross iso-peaks within an activation in each EP signal.

12. The system according to claim 8, wherein the processor is configured to partition the set of EP signals by assigning the EP signals to the clusters based on a width of an activation in each EP signal.

13. The system according to claim 8, wherein the processor is configured to partition the set of EP signals by assigning the EP signals to the clusters based on a peak-to-peak bi-polar voltage of an activation in each EP signal.

14. The system according to claim 8, wherein the processor is configured to partition the set of EP signals by assigning the EP signals to the clusters based on a sharpest slope of an activation in each EP signal.

15. The method according to claim 1 or the system according to claim 8, wherein the similarity measure is one of a linear sum function and a quadratic sum function.

16. The method according to claim 1 or the system according to claim 8, wherein the EP signals are electrograms.

FIG. 1

EP 4 169 446 A1

EP signal type 1

EP signal type 2

EP signal type 3

EP signal type 4

EP signal type 5

EP signal type 6

EP signal type 7

EP signal type 8

EP signal type 9

EP signal type 10

*FIG. 2*

DEFINE DIFFERENT TYPES OF ARRHYTHMIA
TO BE CLUSTERED — 302

DEFINE A "SIMILARITY MEASURE" FOR EACH
TYPE — 304

ACQUIRE SET OF EP SIGNALS — 306

DIVIDE SET INTO CLUSTERS ACCORDING TO
DEFINED TYPES AND MEASURES — 308

DISPLAY REPRESENTATIVE
GRAPHS OF THE EP SIGNALS ACCORDING TO
ARRHYTHMIA TYPES — 310

*FIG. 3*

Europäisches
Patentamt

European
Patent Office

Office européen
des brevets

# EUROPEAN SEARCH REPORT

**Application Number**

**EP 22 20 2345**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/059763 A1 (SHAKUR RAMEEN [GB] ET AL) 28 February 2019 (2019-02-28)<br><br>* paragraphs [0035], [0107], [0112] – [0115], [0120], [0132], [0148], [0163], [0200] *<br>----- | 1-3,5, 7-10,12, 14,15 | INV.<br>A61B5/287<br>A61B5/318<br>A61B5/349<br>A61B5/361<br>A61B5/363<br>A61B5/00 |
| X | WO 2019/212833 A1 (UNIV LELAND STANFORD JUNIOR [US]) 7 November 2019 (2019-11-07)<br>* paragraphs [0100] – [0103], [0109], [0113], [0129] – [0131], [0139], [0151], [0162], [0191] *<br>----- | 1,4,6,8, 11,13,16 | |
| X | ZETAO LIN ET AL: "Algorithm for Clustering Analysis of ECG Data", ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 September 2005 (2005-09-01), pages 3857-3860, XP010906620, DOI: 10.1109/IEMBS.2005.1615302 ISBN: 978-0-7803-8741-6 * page 3858, left-hand column, line 34 – page 3859, right-hand column, line 29 *<br>----- | 1,8 | |

**TECHNICAL FIELDS SEARCHED (IPC)**

A61B

The present search report has been drawn up for all claims

| Place of search | Date of completion of the search | Examiner |
|---|---|---|
| The Hague | 24 February 2023 | Kowalczyk, Szczepan |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
.......................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 22 20 2345

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2023

| Patent document cited in search report | | Publication date | Patent family member(s) | | Publication date |
|---|---|---|---|---|---|
| US 2019059763 A1 | | 28-02-2019 | EP | 3672474 A1 | 01-07-2020 |
| | | | US | 2019059763 A1 | 28-02-2019 |
| | | | WO | 2019038109 A1 | 28-02-2019 |
| WO 2019212833 A1 | | 07-11-2019 | AU | 2019262858 A1 | 10-12-2020 |
| | | | CA | 3098979 A1 | 07-11-2019 |
| | | | CN | 112639990 A | 09-04-2021 |
| | | | EP | 3788588 A1 | 10-03-2021 |
| | | | JP | 2021521964 A | 30-08-2021 |
| | | | US | 2021236053 A1 | 05-08-2021 |
| | | | WO | 2019212833 A1 | 07-11-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9737227 B
- US 20100280400
- US 20040176697
- US 8456182 B

**Non-patent literature cited in the description**

- **KRIEGEL et al.** Density-based clustering. *WIREs Data Mining and Knowledge Discovery,* May 2011, vol. 1 (3), 231-240